# EUROPEAN PATENT SPECIFICATION

(11) **EP 3 825 910 B1**
(45) Date of publication and mention of the grant of the patent: **03.05.2023**
(21) Application number: 20209071.8
(22) Date of filing: 20.11.2020
(51) Int. Cl.: A61B 8/08, G06N 20/00, G06V 10/82, G06V 10/44

(54) **METHOD AND APPARATUS OF INTELLIGENT ANALYSIS FOR LIVER TUMOUR**
VERFAHREN UND VORRICHTUNG ZUR INTELLIGENTEN ANALYSE VON LEBERTUMOREN
PROCÉDÉ ET APPAREIL D'ANALYSE INTELLIGENTE DE TUMEURS DU FOIE

(30) Priority: 21.11.2019 TW 108142298
(43) Date of publication of application: 26.05.2021
(73) Proprietor: Nien, Hsiao-Ching, Taipei City 10041 (TW); YUAN High-Tech Development Co. Ltd., Tapei City 100515 (TW)
(72) Inventor: Nien, Hsiao-Ching, 10041 Taipei City (TW); Lin, Ta-Hsiang, 10050 Taipei City (TW); Chou, Pei-Lien, 10050 Taipei City (TW)
(74) Representative: Lang, Christian

(56) References cited:
- CN-A- 111 243 042
- CN-A- 111 539 930
- DE-A1-102021 124 341
- YASSER M KADAH ET AL: "Classification Algorithms for Quantitative Tissue Characterization of Diffuse Liver Disease from Ultrasound Images", IEEE TRANSACTIONS ON MEDICAL IMAGING, IEEE SERVICE CENTER, PISCATAWAY, NJ, US, vol. 15, no. 4, 1 August 1996 (1996-08-01) , XP011035565, ISSN: 0278-0062

## Description

The present invention relates to apparatus for analysing a liver tumour more particularly, to coordinating scanning acoustic tomography (SAT) with a deep learning algorithm to determine the risk probability of malignant liver tumour, where, with a SAT image, a help to a doctor or ultrasound technician is immediately obtained for determining the risk probability of malignance of a liver tumour and a base of reference is further provided for diagnosing the liver tumour type.

US2018276821 A1 discloses the use of user inputs ultrasound images of a group of cases and the type of liver cancer in each case as training data and a process by which the lesion type of the training data is judged.

Liver cancer is the fourth largest cause of death worldwide. The most common causes of liver cancer in Asia are B-type and C-type hepatitis viruses and aflatoxin. The C-type hepatitis virus is a common cause in the United States and Europe. The liver cancers caused by steatohepatitis, diabetes, and triglyceride have become increasingly serious.

Surgery is currently the most direct method for treating liver cancers. However, early liver cancer diagnoses and postoperative patient-related prognostic indicators are also very important. A patient having liver cancer confirmed by early diagnosis usually have more treatment options, where the treatment efficacy is shown by an improved survival rate of patients. Therefore, regular inspection and early diagnosis and treatment are the keys to improve the quality of life and to prolong the survival rate of patients.

In addition to early diagnoses including liver function blood test, B-type and C-type hepatitis virus, and A-type fetoprotein, abdominal ultrasound is an important test for liver disease, as studies have indicated. An early study denoted that the liver blood tests of 1/3 patients with small Hepatocellular carcinoma (HCC) remained normal indexes for A-type fetoprotein. Ultrasound examination must be complemented for early detection of liver cancer. Furthermore, abdominal ultrasound examination has the advantageous features of being quick, easy and does not expose the patient to radiation, making it an important tool for screening liver cancer.

The diagnosis of liver cancer is different from those of other cancers. Its confirmation does not require biopsy, but is directly obtained through imaging diagnosis like abdominal ultrasound, computed tomography (CT), and magnetic resonance imaging (MRI), etc. Its sensitivity and specificity are 0.78-0.73 and 0.89-0.93, 0.84-0.83 and 0.99-0.91, and 0.83 and 0.88, respectively.

SAT is convenient, but has its own limitations. For example, operator experience, patient obesity, existence of liver fibrosis or cirrhosis, etc. affect the detail and accuracy of information that can be determined from an ultrasound image. Therefore, when malignancy is detected out through SAT, a second imaging detection is also arranged, like CT or assisted diagnosis of MRI. Yet, these two detections have expensive costs for health care and lengthy examination schedules; and CT has consideration on more radiation exposure.

Hence, the prior arts do not fulfil all users' requests on actual use.

The main purpose of the present invention is to coordinate SAT with a deep learning algorithm to determine the nature of a liver tumour, where the method of the invention is able to achieve an accuracy rate reaching as high as 86%, similar to that of CT or MRI, and thus provides physicians with radiation-free and safe SAT to rapidly and accurately diagnose liver tumour categories.

To achieve the above purpose, the present disclosure includes a method of intelligent analysis (IA) for liver tumour, comprising steps of: (a) first step: providing a device of scanning acoustic tomography (SAT) to scan an area of liver of an examinee from an external position to obtain an ultrasonic image of a target liver tumour of the examinee; (b) second step: obtaining a plurality of existing ultrasonic reference images of benignant and malignant liver tumours; (c) third step: obtaining a plurality of liver tumour categories from the existing ultrasonic reference images based on the shading and shadowing areas of the existing ultrasonic reference images to mark a plurality of tumour pixel areas in the existing ultrasonic reference images and identify the liver tumour categories of the tumour pixel areas; (d) fourth step: obtaining the tumour pixel areas in the ultrasonic reference images to train a categoriser model with the coordination of a deep learning algorithm; and (e) fifth step: processing an analysis of the ultrasonic image of the target liver tumour of the examinee with the categoriser model to provide the analysis to a clinician to determine the target liver tumour a liver tumour category and predict a risk probability of malignance of the target liver tumour.

The present invention will now be described by way of example with reference to the following drawings in which:
- Figure 1: is a flow diagram showing a preferred embodiment of a method of intelligent analysis (IA) for liver tumour; and
- Figure 2: is a schematic of apparatus used to carry out the method.

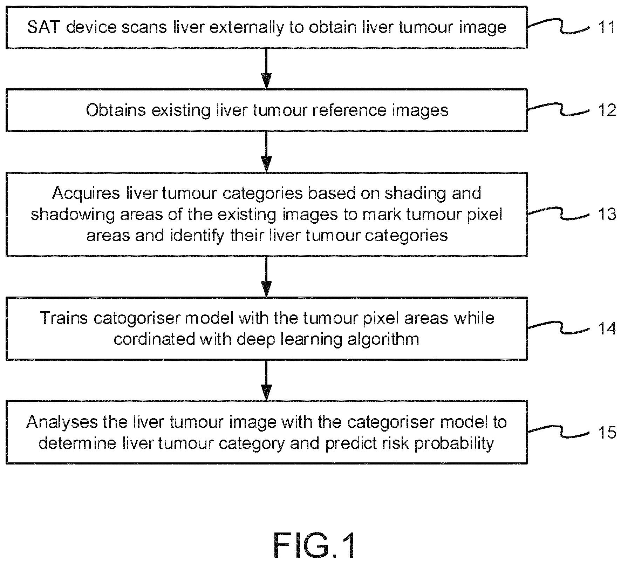

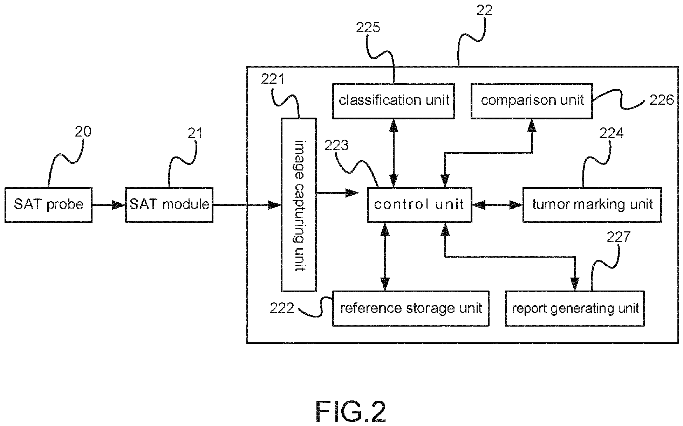

Figures 1 and 2 show a method an apparatus for intelligent analysis (IA) for liver tumour, comprising the following steps:
(a) First step 11: A device of scanning acoustic tomography (SAT) is provided to scan an area of liver of an examinee from an external position to obtain an ultrasonic image of a target liver tumour of the examinee.
(b) Second step 12: A plurality of existing ultrasonic reference images of benignant and malignant liver tumours are obtained.
(c) Third step 13: Based on the shading and shadowing areas of the existing ultrasonic reference images, a plurality of liver tumour categories of the existing ultrasonic reference images are acquired to mark a plurality of tumour pixel areas in the existing ultrasonic reference images and identify the liver tumour categories of the tumour pixel areas.
(d) Fourth step 14: The tumour pixel areas in the ultrasonic reference images is used to train a categoriser model with the coordination of a deep learning algorithm
(e) Fifth step 15: An analysis of the ultrasonic image of the target liver tumour of the examinee is processed with the categoriser model to be provided to a clinician to determine the target liver tumour a liver tumour category and predict a risk probability of malignance of the target liver tumour. Thus, a novel method of IA for liver tumour is obtained and disclosed herein, whereas the claimed invention is defined in the claims.

The present invention uses an apparatus, comprising a SAT module 21 and an analysis module 22.

The SAT module 21 has an ultrasound probe 20.

The analysis module 22 connects to the SAT module 21 and comprises an image capturing unit 221, a reference storage unit 222, a control unit 223, a tumour marking unit 224, a classification unit 225, a comparison unit 226, and a report generating unit 227. Therein, the control unit 223 is a central processing unit (CPU) processing calculations, controls, operations, encoding, decoding, and driving commands with/to the image capturing unit 221, the reference storage unit 222, the tumour marking unit 224, the classification unit 225, the comparison unit 226, and the report generating unit 227.

For applications, the present invention is practiced in a computer. The control unit 222 is a CPU of the computer; the tumour marking unit 224, the classification unit 225, the comparison unit 226, and the report generating unit 227 are programs and stored in a hard disk or a memory of the computer; the image capturing unit 221 is a digital visual interface (DVI) of the computer; the reference storage unit 222 is a hard drive; and the computer further comprises a screen, a mouse, and a keyboard for related input and output operations. Or, the present invention can be implemented in a server.

On using the present invention, an ultrasound probe 20 of a SAT module 21 provides emission of SAT to an examinee from an external position corresponding to an area of liver to obtain an ultrasonic image of a target liver tumour of the examinee. During scanning, a doctor may perceive at least one ultrasound image of a suspected tumour to be selected as an ultrasonic image of a target liver tumour.

By using an image capturing unit 221, an analysis module 22 obtains the ultrasound image of the target liver tumour of the examinee, where the image is formed through imaging with the SAT module 21. A reference storage unit 222 stores a plurality of existing ultrasonic reference images of benignant and malignant liver tumours. A program is stored in an analysis module 22, where, on executing the program by a control unit 223, the program determines a liver tumour category to a clinician and predict a risk probability of malignance of the target liver tumour. The program comprises a tumour marking unit 224, a classification unit 225, a comparison unit 226, and a report generating unit 227.

The tumour marking unit 224 obtains coefficients and/or parameters coordinated with empirical data to automatically mark pixel tumour areas in the ultrasonic reference images and identify a plurality of liver tumour categories. For example, the tumour marking unit 224 may process marking based on physician experiences. The classification unit 225 obtains the pixel tumour areas in the ultrasonic reference images to process training by using a deep learning algorithm to build a categoriser model. The comparison unit 226 analyses the ultrasonic image of the target liver tumour with the categoriser model to provide the clinician for determining the nature of the liver tumour of the examinee and further predicting a risk probability of malignance of the target liver tumour of the examinee. At last, the comparison unit 226 determines the liver tumour category and predicts the risk probability of malignance of the liver tumour by the clinician for the examinee to be inputted to the report generating unit 227 to produce a diagnostic report for assisting the physician in determining the nature of the liver tumour.

Thus, the present invention uses the abundant experiences of abdominal ultrasound specialists as a base to mark a pixel area of liver tumour in an ultrasound image. The parameters and coefficients of such empirical data are obtained for processing training by using the deep learning algorithm to establish the categoriser model having an accuracy ratio up to 86 percent (%). Hence, with the SAT image, a help to the doctor or ultrasound technician is immediately obtained through the present invention for determining the risk probability of malignance of the liver tumour and a base of reference is further provided for diagnosing the liver tumour category.

To sum up, the present disclosure includes a method of IA for liver tumour, where SAT is coordinated with a deep learning algorithm to determine the risk probability of malignant liver tumour; by using coefficients and/or parameters coordinated with empirical data, pixel tumour areas in ultrasonic reference images are marked out to obtain a categoriser model having an accuracy up to 86% through the deep learning algorithm; and, thus, physicians are assisted with radiation-free and safe SAT to rapidly and accurately diagnose liver tumour categories.

The preferred embodiment herein disclosed is not intended to unnecessarily limit the scope of the invention, which is defined in the claims.

## Claims

1. A computer adapted to carry out the following method: a method of intelligent analysis (IA) for liver tumour, comprising steps of:
providing a device of scanning acoustic tomography (SAT) comprising a SAT module (21) and an analysis module (22) for scanning of an area of liver of an examinee (11) from an external position to obtain an ultrasonic image of a target liver tumour of said examinee by an image capturing unit (221) of the analysis module (22);
obtaining a plurality of existing ultrasonic reference images of benignant and malignant liver tumours (12);
obtaining a plurality of liver tumour categories from said existing ultrasonic reference images based on the shading and shadowing areas of said existing ultrasonic reference images to mark a plurality of tumour pixel areas in said existing ultrasonic reference images and identify said liver tumour categories of said tumour pixel areas (13) by a tumour marking unit (224) of the analysis module (22); obtaining said tumour pixel areas in said ultrasonic reference images to train a categoriser model with the coordination of a deep learning algorithm (14) by a classification unit (225) of the analysis module (22); and
processing an analysis of the ultrasonic image of said target liver tumour of said examinee with said categoriser model to provide said analysis by a comparison unit (226) of the analysis module (22), wherein the computer is adapted to determine a liver tumour category and to predict a risk probability of malignance of the target liver tumour.

2. The computer according to claim 1, wherein said SAT module (21) has an ultrasound probe to provide an emission of SAT to an examinee from an external position corresponding to an area of liver to obtain an ultrasonic image of a target liver tumour of said examinee (11).

3. The computer according to claim 1 or 2, wherein said analysis module (22) comprises a control unit (223); a reference storage unit (222) connected with said control unit; and a report generating unit (227) connected with said control unit (223), wherein the image capturing unit (221) is connected with said control unit (223); the tumour marking unit (224) is connected with said control unit (223), the classification unit (225) is connected with said control unit (223) and wherein the comparison unit (226) is connected with said control unit (223).

4. The computer according to claim 3, wherein said control unit (223) is a central processing unit and processes calculations, controls, operations, encoding, decoding, and driving commands to said image capturing unit (221), said reference storage unit (222), said tumour marking unit (224), said classification unit (225), said comparison unit (226), and said report generating unit (227).

5. The computer according to claim 3 or 4, wherein said image capturing unit (221) is a digital visual interface (DVI).

6. The computer according to claim 3, 4 or 5, wherein said reference storage unit (222) stores a plurality of existing ultrasonic reference images of benignant and malignant liver tumours; and said reference storage unit is a hard drive.

7. The computer according to any claim 3-6, wherein said tumour marking unit (224) obtains a plurality of liver tumour categories from said existing ultrasonic reference images based on the shading and shadowing areas of said existing ultrasonic reference images to mark a plurality of tumour pixel areas in said existing ultrasonic reference images and identify said liver tumour categories of said tumour pixel areas.

8. The computer according to claim 7, wherein said tumour marking unit (224) obtains coefficients and/or parameters coordinated with empirical data to automatically mark said tumour pixel areas in said ultrasonic reference images.

9. The computer according to any claim 3-8, wherein said classification unit (224) obtains said tumour pixel areas in said ultrasonic reference images to train a categoriser model with the coordination of a deep learning algorithm.

10. The computer according to any claim 3-9, wherein said comparison unit analyses an ultrasonic image, which is of said target liver tumour of an examinee obtained by said image capturing unit, with a categoriser model, which is built by said classification unit (225).

11. The computer according to any previous claim, wherein said liver tumour categories comprise benignant liver tumour categories and malignant liver tumour categories.

12. Apparatus for analysing a liver tumour, the apparatus adapted to
receive an ultrasonic image of a target liver tumour of an examinee;
receive a plurality of existing ultrasonic reference images of benignant and malignant liver tumours;
obtain a plurality of liver tumour categories from said existing ultrasonic reference images based on the shading and shadowing areas of said existing ultrasonic reference images;
mark a plurality of tumour pixel areas in said existing ultrasonic reference images and identify said liver tumour categories of said tumour pixel areas;
obtain said tumour pixel areas in said ultrasonic reference images to train a categoriser model with the coordination of a deep learning algorithm; and
processing an analysis of the ultrasonic image of said target liver tumour of said examinee with said categoriser model to provide said analysis, wherein the apparatus is adapted to determine a liver tumour category and to predict a risk probability of malignance of the target liver tumour.

## Patentansprüche

1. Computer, der geeignet ist, das folgende Verfahren auszuführen: ein Verfahren zur intelligenten Analyse (IA) von Lebertumoren, das die folgenden Schritte umfasst:
Bereitstellen einer Vorrichtung zur akustischen Scanning-Tomographie (SAT), die ein SAT-Modul (21) und ein Analysemodul (22) zum Scannen eines Leberbereichs einer Untersuchungsperson (11) von einer externen Position aus umfasst, um ein Ultraschallbild eines Ziel-Lebertumors der Untersuchungsperson durch eine Bilderfassungseinheit (221) des Analysemoduls (22) zu erhalten;
Erhalten einer Vielzahl von vorhandenen Ultraschallreferenzbildern von gutartigen und bösartigen Lebertumoren (12);
Erhalten einer Vielzahl von Lebertumorkategorien aus den vorhandenen Ultraschallreferenzbildern auf der Grundlage der Schattierungs- und Schattenbereiche der vorhandenen Ultraschallreferenzbilder, um eine Vielzahl von Tumorpixelbereichen in den vorhandenen Ultraschallreferenzbildern zu markieren und die Lebertumorkategorien der Tumorpixelbereiche (13) durch eine Tumormarkierungseinheit (224) des Analysemoduls (22) zu identifizieren;
Erhalten der Tumorpixelbereiche in den Ultraschallreferenzbildern, um ein Kategorisierungsmodell mit der Koordination eines Deep-Learning-Algo- rithmus (14) durch eine Klassifizierungseinheit (225) des Analysemoduls (22) zu trainieren; und
Verarbeiten einer Analyse des Ultraschallbildes des Ziel-Lebertumors der Untersuchungsperson mit dem Kategorisierungsmodell, um die Analyse durch eine Vergleichseinheit (226) des Analysemoduls (22) bereitzustellen, wobei der Computer angepasst ist, um eine Lebertumorkategorie zu bestimmen und eine Risikowahrscheinlichkeit der Bösartigkeit des Ziel-Lebertumors vorherzusagen.

2. Computer nach Anspruch 1, wobei das SAT-Modul (21) eine Ultraschallsonde aufweist, um eine SAT-Emission an einer Untersuchungsperson von einer externen Position aus zu liefern, die einem Bereich der Leber entspricht, um ein Ultraschallbild eines Ziel-Lebertumors der Untersuchungsperson (11) zu erhalten.

3. Computer nach Anspruch 1 oder 2, wobei das Analysemodul (22) eine Steuereinheit (223), eine mit der Steuereinheit verbundene Referenzspeichereinheit (222) und eine mit der Steuereinheit (223) verbundene Berichterzeugungseinheit (227) umfasst, wobei die Bilderfassungseinheit (221) mit der Steuereinheit (223) verbunden ist, die Tumormarkierungseinheit (224) mit der Steuereinheit (223) verbunden ist, die Klassifizierungseinheit (225) mit der Steuereinheit (223) verbunden ist und wobei die Vergleichseinheit (226) mit der Steuereinheit (223) verbunden ist.

4. Computer nach Anspruch 3, wobei die Steuereinheit (223) eine zentrale Verarbeitungseinheit ist und Berechnungen, Steuerungen, Operationen, Kodierung, Dekodierung und Steuerbefehle an die Bilderfassungseinheit (221), die Referenzspeichereinheit (222), die Tumormarkierungseinheit (224), die Klassifizierungseinheit (225), die Vergleichseinheit (226) und die Berichterzeugungseinheit (227) verarbeitet.

5. Computer nach Anspruch 3 oder 4, wobei die Bilderfassungseinheit (221) eine digitale visuelle Schnittstelle (DVI) ist.

6. Computer nach einem der Ansprüche 3, 4 oder 5, wobei die Referenzspeichereinheit (222) eine Vielzahl vorhandener Ultraschallreferenzbilder von gutartigen und bösartigen Lebertumoren speichert und die Referenzspeichereinheit eine Festplatte ist.

7. Computer nach einem der Ansprüche 3 bis 6, wobei die Tumormarkierungseinheit (224) eine Vielzahl von Lebertumorkategorien aus den vorhandenen Ultraschallreferenzbildern auf der Grundlage der Schattierungs- und Schattenbereiche der vorhandenen Ultraschallreferenzbilder erhält, um eine Vielzahl von Tumorpixelbereichen in den vorhandenen Ultraschallreferenzbildern zu markieren und die Lebertumorkategorien der Tumorpixelbereiche zu identifizieren.

8. Computer nach Anspruch 7, wobei die Tumormarkierungseinheit (224) Koeffizienten und/oder Parameter erhält, die mit empirischen Daten koordiniert sind, um die Tumorpixelbereiche in den Ultraschallreferenzbildern automatisch zu markieren.

9. Computer nach einem der Ansprüche 3 bis 8, wobei die Klassifizierungseinheit (225) die Tumorpixelbereiche in den Ultraschallreferenzbildern erhält, um ein Kategorisierungsmodell mit der Koordination eines Deep-Learning-Algo- rithmus zu trainieren.

10. Computer nach einem der Ansprüche 3 bis 9, wobei die Vergleichseinheit ein Ultraschallbild des Ziel-Lebertumors einer Untersuchungsperson, das von der Bilderfassungseinheit erhalten wird, mit einem Kategorisierungsmodell analysiert, das von der Klassifizierungseinheit (225) erstellt wird.

11. Computer nach einem der vorhergehenden Ansprüche, wobei die Lebertumorkategorien gutartige Lebertumorkategorien und bösartige Lebertumorkategorien umfassen.

12. Vorrichtung zum Analysieren eines Lebertumors, wobei die Vorrichtung ausgelegt ist zum
Empfangen eines Ultraschallbilds eines Ziel-Lebertumors einer Untersuchungsperson;
Empfangen einer Vielzahl vorhandener Ultraschallreferenzbilder von gutartigen und bösartigen Lebertumoren,
Erhalten einer Vielzahl von Lebertumorkategorien aus den vorhandenen Ultraschallreferenzbildern auf der Grundlage der Schattierungs- und Schattenbereiche der vorhandenen Ultraschallreferenzbilder;
Markieren einer Vielzahl von Tumorpixelbereichen in den vorhandenen Ultraschallreferenzbildern und Identifizieren der Lebertumorkategorien der Tumorpixelbereiche;
Erhalten der Tumorpixelbereiche in den Ultraschallreferenzbildern, um ein Kategorisierungsmodell mit der Koordination eines Deep-Learning-Algo- rithmus zu trainieren; und
Verarbeiten einer Analyse des Ultraschallbildes des Ziel-Lebertumors der Untersuchungsperson mit dem Kategorisierungsmodell, um die Analyse bereitzustellen, wobei die Vorrichtung angepasst ist, um eine Lebertumorkategorie zu bestimmen und eine Risikowahrscheinlichkeit der Bösartigkeit des Ziel-Lebertumors vorherzusagen.

## Revendications

1. Un ordinateur conçu pour mettre en oeuvre le procédé suivant : un procédé d'analyse intelligente (IA) pour une tumeur du foie, comprenant les étapes de :
fourniture d'un dispositif de tomographie acoustique à balayage (SAT) comprenant un module SAT (21) et un module d'analyse (22) pour balayer une zone du foie d'un sujet examiné (11) depuis une position externe pour obtenir une image ultrasonore d'une tumeur du foie cible dudit sujet examiné par une unité de capture d'image (221) du module d'analyse (22) ;
obtention d'une pluralité d'images de référence ultrasonores existantes de tumeurs du foie bénignes et malignes (12) ;
obtention d'une pluralité de catégories de tumeurs du foie à partir desdites images de référence ultrasonores existantes sur la base des zones d'ombrage et d'opacité desdites images de référence ultrasonores existantes pour marquer une pluralité de zones de pixels de tumeur dans lesdites images de référence ultrasonores existantes et identifier lesdites catégories de tumeurs du foie desdites zones de pixels de tumeur (13) par une unité de marquage de tumeur (224) du module d'analyse (22) ; obtention desdites zones de pixels de tumeur dans lesdites images de référence ultrasonores pour former un modèle de catégorisation sous la coordination d'un algorithme d'apprentissage profond (14) par une unité de classification (225) du module d'analyse (22) ; et
traitement d'une analyse de l'image ultrasonore de ladite tumeur du foie cible dudit sujet examiné avec ledit modèle de catégorisation pour fournir ladite analyse par une unité de comparaison (226) du module d'analyse (22), dans lequel l'ordinateur est conçu pour déterminer une catégorie de tumeur du foie et pour prédire une probabilité des risques de malignité de la tumeur du foie cible.

2. L'ordinateur selon la revendication 1,
dans lequel ledit module SAT (21) présente une sonde ultrasonore pour fournir une émission de SAT à un sujet examiné depuis une position externe correspondant à une zone du foie pour obtenir une image ultrasonore d'une tumeur du foie cible dudit sujet examiné (11).

3. L'ordinateur selon la revendication 1 ou 2,
dans lequel ledit module d'analyse (22) comprend une unité de commande (223) ; une unité de stockage de référence (222) connectée à ladite unité de commande ; et une unité de génération de rapport (227) connectée à ladite unité de commande (223), dans lequel l'unité de capture d'image (221) est connectée à ladite unité de commande (223) ; l'unité de marquage de tumeur (224) est connectée à ladite unité de commande (223), l'unité de classification (225) est connectée à ladite unité de commande (223) et dans lequel l'unité de comparaison (226) est connectée à ladite unité de commande (223).

4. L'ordinateur selon la revendication 3,
dans lequel ladite unité de commande (223) est une unité de traitement centrale et traite des calculs, des commandes, des opérations, le codage, le décodage, et des instructions de pilotage à ladite unité de capture d'image (221), ladite unité de stockage de référence (222), ladite unité de marquage de tumeur (224), ladite unité de classification (225), ladite unité de comparaison (226), et ladite unité de génération de rapport (227).

5. L'ordinateur selon la revendication 3 ou 4,
dans lequel ladite unité de capture d'image (221) est une interface visuelle numérique (DVI).

6. L'ordinateur selon la revendication 3, 4 ou 5,
dans lequel ladite unité de stockage de référence (222) stocke une pluralité d'images de référence ultrasonores existantes de tumeurs du foie bénignes et malignes ; et ladite unité de stockage de référence est un disque dur.

7. L'ordinateur selon l'une quelconque des revendications 3 à 6,
dans lequel ladite unité de marquage de tumeur (224) obtient une pluralité de catégories de tumeurs du foie à partir desdites images de référence ultrasonores existantes sur la base des zones d'ombrage et d'opacité desdites images de référence ultrasonores existantes pour marquer une pluralité de zones de pixels de tumeur dans lesdites images de référence ultrasonores existantes et identifier lesdites catégories de tumeurs du foie desdites zones de pixels de tumeur.

8. L'ordinateur selon la revendication 7,
dans lequel ladite unité de marquage de tumeur (224) obtient des coefficients et/ou des paramètres coordonnés avec des données empiriques pour marquer automatiquement lesdites zones de pixels de tumeur dans lesdites images de référence ultrasonores.

9. L'ordinateur selon l'une quelconque des revendications 3 à 8,
dans lequel ladite unité de classification (225) obtient lesdites zones de pixels de tumeur dans lesdites images de référence ultrasonores pour former un modèle de catégorisation sous la coordination d'un algorithme d'apprentissage profond.

10. L'ordinateur selon l'une quelconque des revendications 3 à 9,
dans lequel ladite unité de comparaison analyse une image ultrasonore, qui représente ladite tumeur du foie cible d'un sujet examiné obtenue par ladite unité de capture d'image, avec un modèle de catégorisation, qui est construit par ladite unité de classification (225).

11. L'ordinateur selon une quelconque revendication précédente,
dans lequel lesdites catégories de tumeurs du foie comprennent des catégories de tumeurs du foie bénignes et des catégories de tumeurs du foie malignes.

12. Appareil d'analyse d'une tumeur du foie, l'appareil étant conçu pour recevoir une image ultrasonore d'une tumeur du foie cible d'un sujet examiné ;
recevoir une pluralité d'images de référence ultrasonores existantes de tumeurs du foie bénignes et malignes ;
obtenir une pluralité de catégories de tumeurs du foie à partir desdites images de référence ultrasonores existantes sur la base des zones d'ombrage et d'opacité desdites images de référence ultrasonores existantes ;
marquer une pluralité de zones de pixels de tumeur dans lesdites images de référence ultrasonores existantes et identifier lesdites catégories de tumeurs du foie desdites zones de pixels de tumeur ;
obtenir lesdites zones de pixels de tumeur dans lesdites images de référence ultrasonores pour former un modèle de catégorisation sous la coordination d'un algorithme d'apprentissage profond ; et
traiter une analyse de l'image ultrasonore de ladite tumeur du foie cible dudit sujet examiné avec ledit modèle de catégorisation pour fournir ladite analyse, dans lequel l'appareil est conçu pour déterminer une catégorie de tumeur du foie et pour prédire une probabilité des risques de malignité de la tumeur du foie cible.
